# EUROPÄISCHE PATENTSCHRIFT

(11) **EP 1 526 129 B1**
(45) Veröffentlichungstag und Bekanntmachung des Hinweises auf die Patenterteilung: **23.06.2010**
(21) Anmeldenummer: 04024364.4
(22) Anmeldetag: 13.10.2004
(51) Int. Cl.: C07C 263/10, C07C 265/04, C07C 265/10, C07C 265/12, C07C 265/14

(54) **Verfahren zur Herstellung von Isocyanaten in der Gasphase**
Process for the preparation of isocyanates in the gas phase
Procédé de préparation d'isocyanates en phase gazeuze

(30) Priorität: 23.10.2003 DE 10349504
(43) Veröffentlichungstag der Anmeldung: 27.04.2005
(73) Patentinhaber: Bayer MaterialScience AG, 51368 Leverkusen (DE)
(72) Erfinder: Herold, Heiko, 41470 Neuss (DE); Michele, Volker, Dr., 51065 Köln (DE); König, Werner, 51379 Leverkusen (DE)

(56) Entgegenhaltungen:
- EP-A- 0 065 727
- EP-A- 1 449 826
- DE-A1- 1 792 660
- DE-A1- 2 950 216

## Beschreibung

Die Erfindung betrifft ein Verfahren zur Herstellung von Isocyanaten in der Gasphase, bei dem durch verbesserte Reaktionsführung in Rohrreaktoren durch strömungstechnische Maßnahmen, wie die Erhöhung der Turbulenz, die Durchmischung der Reaktanden deutlich verbessert wird. In der Folge werden die erforderliche Verweilzeit der Reaktanden im Reaktor und somit die nötige Reaktorbaulänge verkürzt und die Bildung polymerer Nebenprodukte vermieden, die zu Anbackungen im Reaktor und Verkürzung der Standzeit der Reaktoren führen.

EP-A-0065727 beschreibt ein Verfahren zur Herstellung von organischen Mono-od Polyisocyanaten.

In der EP-A 0 289 840 wird ein Verfahren zur Herstellung von (cyclo)aliphatischen Diisocyanaten durch Phosgenierung der entsprechenden, dampfförmigen (cyclo)aliphatischen Diamine bei 200°C bis 600°C beschrieben. Phosgen wird in stöchiometrischem Überschuss zugeführt. Die überhitzten Ströme von dampfförmigem (cyclo)aliphatischem Diamin bzw. (cyclo)aliphatischem Diamin-Inertgas-Gemisch einerseits und von Phosgen andererseits werden kontinuierlich in einen zylindrischen Reaktionsraum geleitet, dort miteinander vermischt und zur Reaktion gebracht. Die exotherme Phosgenierreaktion wird unter Aufrechterhaltung einer turbulenten Strömung durchgeführt.

Gasförmige Edukte werden häufig in Rohrreaktoren reaktionstechnisch umgesetzt. Beim Strahlmischerprinzip (Chemie-Ing.-Techn. 44 (1972) S. 1055, Abb. 10) werden dem Rohrreaktor zwei Eduktströme A und B zugeführt, wobei der Eduktstrom A über eine Zentraldüse und der Eduktstrom B über einen Ringraum zwischen Zentraldüse und Rohrreaktorwand zugeführt werden. Die Strömungsgeschwindigkeit des Eduktstroms A ist groß gegenüber der Strömungsgeschwindigkeit des Eduktstroms B. Hierdurch kommt es im Rohrreaktor zu einer Vermischung der Reaktionspartner und in der Folge zu deren Reaktion. Diese Art der Reaktionsführung hat technische Bedeutung bei der Herstellung von aromatischen Diisocyanaten durch Phosgenierung von aromatischen Diaminen in der Gasphase erlangt (z.B. EP-A-0 570 799).

Die bekannten Verfahren erfordern jedoch eine große Baulänge der Reaktoren, da die Vermischung ohne weitergehende Maßnahmen langsam vonstatten geht.

Folge der langsamen Durchmischung der Reaktanden ist die Bildung polymerer Nebenprodukte, die zu Anbackungen bis hin zu Verstopfungen im Reaktor und damit zur Verkürzung der Standzeit der Reaktoren führen. Außerdem führen die größeren Reaktorbaulängen zu erhöhten Investitionskosten.

Die Aufgabe der Erfindung ist es daher, ein Verfahren zur Herstellung von (cyclo)aliphatischen und aromatischen Diisocyanaten durch Phosgenierung entsprechender (cyclo)aliphatischer und aromatischer Diamine in der Gasphase bei hohen Temperaturen zu finden, bei dem die Durchmischung der Reaktanden deutlich schneller abläuft als bei den bisher bekannten Verfahren.

Es wurde nun überraschenderweise gefunden, dass eine Erhöhung der Turbulenz des Eduktstromes in der Zentraldüse einen positiven Einfluss auf die Durchmischung der Reaktanden und damit auf die Gasphasenreaktion insgesamt hat. Als Folge der besseren Durchmischung nimmt die Neigung zur Nebenproduktbildung ab und die erforderliche Verweilzeit und somit Reaktorbaulänge sinken deutlich. Somit können die Nachteile der Verfahren des Standes der Technik deutlich reduziert werden, wenn die Eduktströme den erfindungsgemäßen, nachstehend näher beschriebenen Maßnahmen unterworfen werden.

Gegenstand der Erfindung ist ein Verfahren zur Herstellung von Diisocyanaten und Triisocyanaten der allgemeinen Formel (I)

R(NCO)ₙ (I),

in welcher
- R: für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
- n: für die Zahl 2 oder 3 steht,
durch Phosgenierung der entsprechenden Diamine und/oder Triamine der allgemeinen Formel (II) in der Gasphase

R(NH₂)ₙ (II),

in welcher
- R: für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
- n: für die Zahl 2 oder 3 steht,
dadurch gekennzeichnet, dass die Phosgenierung in einem Rohrreaktor durchgeführt wird, der eine Zentraldüse und einen Ringraum zwischen der Zentraldüse und der Wand des Rohrreaktors enthält, wobei die Zentraldüse in dem Rohrreaktor zentriert ist, wobei die Zentraldüse mit einem Einlass für einen der Eduktströme verbunden ist und in dem Ringraum der Einlass für einen zweiten Eduktstrom angeordnet ist, wobei in der Zentraldüse eine Turbulenz erzeugt wird,
wobei der die Diamine und/oder Triamine enthaltende Eduktstrom dem Rohrreaktor über die Zentraldüse zugeführt wird, und der Phosgen enthaltende Eduktstrom dem Rohrreaktor über den Ringraum zugeführt wird. Der Turbulenzgrad der Strömung in der Zentraldüse wird bevorzugt. durch Einbauelemente erhöht.

In einer alternativen Ausführungsform des erfindungsgemäßen Verfahrens werden der die Diamine und/oder Triamine enthaltende Eduktstrom und der Phosgen enthaltende Eduktstrom vertauscht, so dass der die Diamine und/oder Triamine enthaltende Eduktstrom dem Rohrreaktor über den Ringraum und der Phosgen enthaltende Eduktstrom dem Rohrreaktor über die Zentraldüse zugeführt wird.

Vorzugsweise werden eine oder mehrere schräg in die Strömung eingebrachte runde oder eckige Platten oder eine Spiralwendel als turbulenzerhöhende Einbauelemente in der Zentraldüse verwendet.

Aufgabe der Schrägplatte oder der Kombination aus mehreren Schrägplatten ist es, den Turbulenzgrad in der Zentraldüse zu erhöhen.

Aufgabe der Spiralwendel ist es, in der Strömung in der Zentraldüse den Turbulenzgrad zu erhöhen und die Strömung zu verdrallen, um zentrifugale Effekte zur Unterstützung der Vermischung von Innen- und Außenströmung zu nutzen.

Durch das erfindungsgemäße Verfahren gelingt es, die Mischstrecke von über den Ringraum und über die Zentraldüse zugeführten Eduktströmen um mindestens 50 %, gegenüber dem Vergleichswert ohne turbulenzerzeugende Einbauten zu verkürzen.

Im erfindungsgemäßen Verfahren werden Diisocyanate und/oder Triisocyanate aus den entsprechenden Diaminen und/oder Triaminen hergestellt.

Vorzugsweise werden im erfindungsgemäßen Verfahren Diisocyanate durch Phosgenierung der entsprechenden Diamine hergestellt.

Als Triisocyanat der Formel (I) wird im erfindungsgemäßen Verfahren vorzugsweise 1,8-Diisocyanato-4-(isocyanatomethyl)octan, Triisocyanatononan (TIN), hergestellt.

Typische Beispiele geeigneter aliphatischer Diamine sind z.B. in EP-A 0 289 840, typische Beispiele geeigneter aliphatischer Triamine sind z.B. in EP-A 749 958 genannt. Diese Diamine sind geeignet für die Herstellung der entsprechenden Diisocyanate oder Triisocyanate nach dem erfindungsgemäßen Verfahren.

Bevorzugt werden Isophorondiamin (IPDA), Hexamethylendiamin (HDA) und Bis(paminocyclohexyl)methan.

Typische Beispiele geeigneter aromatischer Diamine sind die reinen Isomeren oder die Isomerengemische des Diaminobenzols, des Diaminotoluols, des Diaminodimethylbenzols, des Diaminonaphthalins sowie des Diaminodiphenylmethans, bevorzugt sind 2,4/2,6-Toluylendiamin-Gemische der Isomerenverhältnisse 80/20 und 65/35 oder das reine 2,4-Toluylendiamin-Isomere.

Als Triamin wird vorzugsweise 1,8-Diamino-4-(aminomethyl)octan, Triaminononan eingesetzt.

Die Ausgangsamine der Formel (II) werden dem Reaktor gasförmig zugeführt und gegebenenfalls vor der Durchführung des erfindungsgemäßen Verfahrens verdampft und bevorzugt auf 200°C bis 600°C, besonders bevorzugt 250°C bis 450°C erhitzt und gegebenenfalls verdünnt mit einem Inertgas wie N₂, Ne, He, Ar oder mit den Dämpfen eines inerten Lösungsmittels dem Reaktor zugeführt. Das Phosgen wird dem Rohrreaktor in stöchiometrischem Überschuss und bei 200°C bis 600°C zugeführt. Bei Einsatz der aliphatischen Diamine beträgt der molare Phosgenüberschuss bezogen auf eine Aminogruppe bevorzugt zwischen 25 % und 250 %, bei Einsatz der aliphatischen Triamine bevorzugt zwischen 50 % und 350 %. Beim Einsatz aromatischer Diamine beträgt der molare Phosgenüberschuss bezogen auf eine Aminogruppe bevorzugt zwischen 150 % und 300 %.

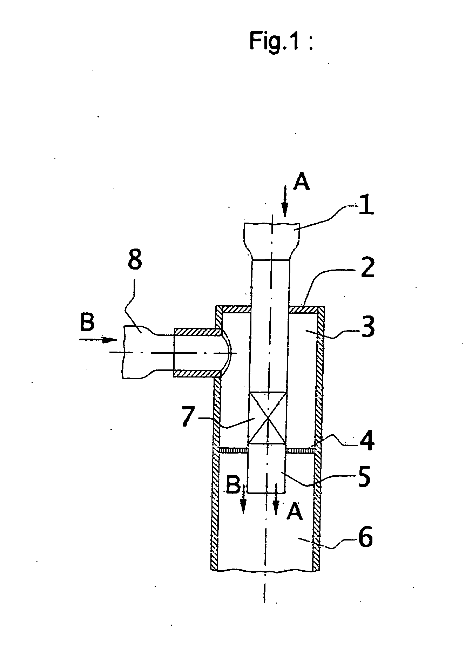

Im Folgenden wird die Erfindung beispielhaft anhand der Fig. 1 erläutert. Der Eduktstrom A (Diamin und / oder Triamin) strömt über den Einlass 1 und die Zentraldüse 5 in den Rohrreaktor 6.

Die Zentraldüse 5 wird von dem Deckel 2 und dem Halter 4 in Position gehalten und ist auf die Rotationsachse des Rohrreaktors 6 zentriert. In der Zentraldüse befinden sich ein oder mehrere turbulenzerzeugende Elemente 7.

Der Eduktstrom B (Phosgen) strömt durch den Einlass 8 in den Ringraum 3 des Rohrreaktors 6.

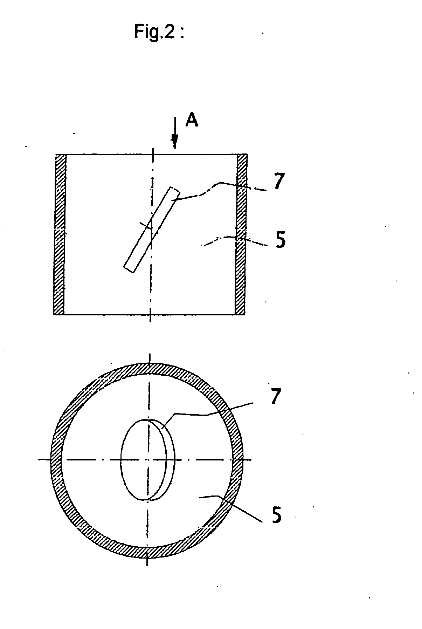

Fig. 2 zeigt eine als Turbulenzerzeuger 7 bevorzugte Schrägplatte.

Fig. 3 zeigt ein als Turbulenzerzeuger 7 bevorzugtes Wendelelement.

### Beispiel

Einem Modell-Rohrreaktor gemäß Fig. 1 (Länge 2000 mm, Innendurchmesser des Außenrohrs 172 mm, Innendurchmesser des Innenrohrs 54 mm) wird als Edukt A und B jeweils Gas zugeführt, wobei das Gas der Innenströmung (Edukt A) durch Zugabe von Aerosolen geseedet wird.

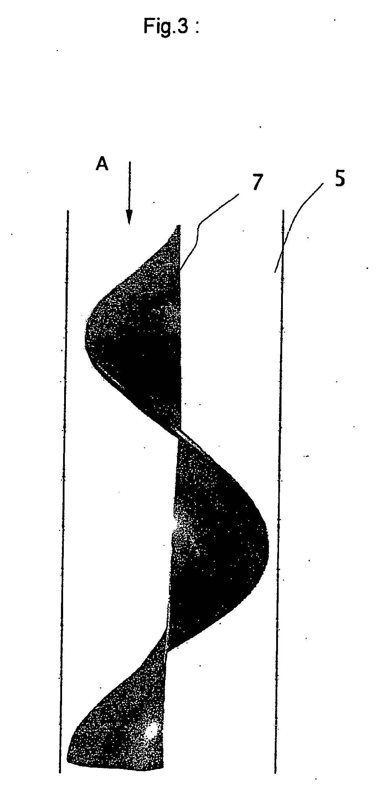

Der Versuch wird zum einen erfindungsgemäß durchgeführt mit einer Spiralwendel gemäß Fig. 3 als turbulenzerzeugendes Element in der Zentraldüse; die Länge der Spiralwendel betrug 135 mm, ihr Durchmesser 54 mm, die Verdrehung 360°.

Zum anderen wird der Versuch vergleichsweise ohne turbulenzerzeugende Einbauten in der Zentraldüse durchgeführt.

Stromabwärts der Mündung der Zentraldüse kann nun die Vermischung von Innen- und Außenströmung anhand der radialen Verteilung der Aerosole der Innenströmung visuell beurteilt werden. Eine vollständige Mischung von Innen- und Außenströmung wird als erreicht angesehen, wenn die Aerosole aus der Innenströmung die Wand des Außenrohrs erreicht haben. Die axiale Länge des Weges im Rohrreaktor von der Mündung der Zentraldüse bis zu diesem Punkt wird im folgenden als Mischungsstrecke bezeichnet.

Bei dem als Vergleichsbeispiel durchgeführten Versuch beträgt die Mischungsstrecke 1200 mm. Bei dem erfindungsgemäß durchgeführten Versuch mit der Spiralwendel als turbulenzerzeugendem Einbauelement beträgt die Mischungsstrecke lediglich 500 mm. Somit verkürzt sich die Mischungsstrecke beim erfindungsgemäßen Verfahren auf 42 % der ursprünglichen Länge.

## Patentansprüche

1. Verfahren zur Herstellung von Diisocyanaten und Triisocyanate der allgemeinen Formel (I)
R(NCO)ₙ (I),
in welcher
R für einen (cyclo)aliphatischen oder aromatischen Kohlenwasserstoffrest mit bis zu 15 Kohlenstoffatomen, vorzugsweise 4 bis 13 Kohlenstoffatomen, steht, mit der Maßgabe, dass zwischen zwei NCO-Gruppen mindestens 2 Kohlenstoffatome angeordnet sind und
n für die Zahl 2 oder 3 steht,
durch Phosgenierung der entsprechenden Diamine und/oder Triamine der allgemeinen Formel (II) in der Gasphase
R(NH₂)ₙ (II),
in welcher
R für einen (cyclo)aliphatischen oder aromatischen Kohlenwasserstoffrest mit bis zu 15, vorzugsweise 4 bis 13 Kohlenstoffatomen steht, mit der Maßgabe, dass zwischen zwei Aminogruppen mindestens zwei Kohlenstoffatome angeordnet sind und
n für die Zahl 2 oder 3 steht,
wobei die Phosgenierung in einem Rohrreaktor (6) durchgeführt wird, der eine Zentraldüse (5) und einen Ringraum (3) zwischen der Zentraldüse (5) und der Wand des Rohrreaktors (6) enthält, **dadurch gekennzeichnet, dass** in der Zentraldüse eine Turbulenz erzeugt wird und insbesondere die Turbulenz der in der Zentraldüse (5) geführten Strömung durch ein oder mehrere turbulenzerzeugende Einbauelemente (7) erhöht wird und wobei der die Diamine und/oder Triamine enthaltende Eduktstrom dem Rohrreaktor (6) über die Zentraldüse (5) zugeführt wird, und der Phosgen enthaltende Eduktstrom dem Rohrreaktor (6) über den Ringraum (3) zugeführt wird.

2. Verfahren nach Anspruch 1, bei dem der die Diamine und / oder Triamine enthaltende Eduktstrom und der Phosgen enthaltende Eduktstrom vertauscht sind, so dass der die Diamine und / oder Triamine enthaltende Eduktstrom dem Rohrreaktor (6) über den Ringraum (3) und der Phosgen enthaltende Eduktstrom dem Rohrreaktor (6) über die Zentraldüse (5) zugeführt wird.

3. Verfahren nach Anspruch 1 oder 2, **dadurch gekennzeichnet, dass** als turbulenzerzeugende Elemente runde oder eckige Schrägplatten verwendet werden.

4. Verfahren nach Anspruch 1 oder 2, **dadurch gekennzeichnet, dass** als turbulenzerzeugendes Element eine Wendel (7) verwendet wird.

5. Verfahren nach einem der Ansprüche 1 bis 4, **dadurch gekennzeichnet, dass** Diisocyanate durch Phosgenierung der entsprechenden Diamine hergestellt werden.

6. Verfahren nach einem der Ansprüche 1 und 3 bis 5, **dadurch gekennzeichnet, dass** dem Rohrreaktor (6) dampfförmige (cyclo)aliphatische und / oder aromatische Diamine bei 200°C bis 600°C über die Zentraldüse (5) zugeführt werden und Phosgen dem Rohrreaktor (6) in stöchiometrischem Überschuss bei 200°C bis 600°C über den Ringraum (3) zugeführt wird.

7. Verfahren nach einem der Ansprüche 1 bis 6, **dadurch gekennzeichnet, dass** Isophorondiamin (IPDA) oder Hexamethylendiamin (HDA) oder Bis(p-aminocyclohexyl)methan als Diamine eingesetzt werden.

8. Verfahren nach einem der Ansprüche 1 bis 6, **dadurch gekennzeichnet, dass** 2,4/2,6-Toluylendiamin-Gemische der Isomerenverhältnisse 80/20 und 65/35 oder das reine 2,4-Toluylendiamin-Isomere als Diamine eingesetzt werden.

9. Verfahren nach einem der Ansprüche 1 bis 8, **dadurch gekennzeichnet, dass** Triisocyanatnonan hergestellt wird.

## Claims

1. Process for the preparation of diisocyanates and triisocyanates of general formula (I):
R(NCO)ₙ (I)
in which
R is a (cyclo)aliphatic or aromatic hydrocarbon radical having up to 15 carbon atoms, preferably 4 to 13 carbon atoms, with the proviso that there are at least 2 carbon atoms between two NCO groups, and
n is the number 2 or 3,
by gas-phase phosgenation of the corresponding diamines and/or triamines of general formula (II):
R(NH₂)ₙ (II)
in which
R is a (cyclo)aliphatic or aromatic hydrocarbon radical having up to 15 carbon atoms, preferably 4 to 13 carbon atoms, with the proviso that there are at least two carbon atoms between two amino groups, and
n is the number 2 or 3,
wherein the phosgenation is carried out in a tubular reactor (6) containing a central nozzle (5) and an annular chamber (3) between the central nozzle (5) and the wall of the tubular reactor (6), **characterized in that** a turbulence is generated in the central nozzle and, in particular, the turbulence of the stream fed into the central nozzle (5) is increased by one or more turbulence-generating baffles (7), and wherein the educt stream containing the diamines and/or triamines is fed into the tubular reactor (6) via the central nozzle (5) and the educt stream containing the phosgene is fed into the tubular reactor (6) via the annular chamber (3).

2. Process according to Claim 1 wherein the educt stream containing the diamines and/or triamines and the educt stream containing the phosgene are swapped so that the educt stream containing the diamines and/or triamines is fed into the tubular reactor (6) via the annular chamber (3) and the educt stream containing the phosgene is fed into the tubular reactor (6) via the central nozzle (5).

3. Process according to Claim 1 or 2, **characterized in that** circular or polygonal inclined plates are used as the turbulence-generating baffles.

4. Process according to Claim 1 or 2, **characterized in that** a helix (7) is used as the turbulence-generating baffle.

5. Process according to one of Claims 1 to 4, **characterized in that** diisocyanates are prepared by phosgenation of the corresponding diamines.

6. Process according to one of Claims 1 and 3 to 5,
**characterized in that** vaporous (cyclo)aliphatic and/or aromatic diamines are fed into the tubular reactor (6) at 200°C to 600°C via the central nozzle (5) and phosgene is fed into the tubular reactor (6) in stoichiometric excess at 200°C to 600°C via the annular chamber (3).

7. Process according to one of Claims 1 to 6, **characterized in that** the diamine used is isophoronediamine (IPDA), hexamethylenediamine (HDA) or bis(p-aminocyclohexyl)methane.

8. Process according to one of Claims 1 to 6, **characterized in that** the diamines used are 2,4/2,6-toluylenediamine mixtures with isomer ratios of 80/20 and 65/35 or the pure 2,4-toluylenediamine isomer.

9. Process according to one of Claims 1 to 8, **characterized in that** triisocyanatononane is prepared.

## Revendications

1. Procédé pour la préparation de diisocyanates et de triisocyanates de la formule générale (I)
R(NCO)ₙ (I),
dans laquelle
R représente un reste hydrocarboné (cyclo)aliphatique ou aromatique avec jusqu'à 15 atomes de carbone, de préférence de 4 à 13 atomes de carbone, à condition que sont disposés entre deux groupes NCO, aux moins deux atomes de carbone et
n représente le nombre de 2 ou de 3,
par phosgénation des diamines et/ou triamines correspondantes de la formule générale (II) en phase vapeur.
R(NH₂)ₙ (II),
dans laquelle
R représente un reste hydrocarboné (cyclo)aliphatique ou aromatique avec jusqu'à 15, de préférence de 4 à 13 atomes de carbone, à condition que sont disposés entre deux groupes amino au moins deux atomes de carbone et
n représente le nombre de 2 ou de 3,
la phosgénation étant réalisée dans un réacteur tubulaire (6), lequel contient une buse centrale (5) et un espace annulaire (3) entre la buse centrale (5) et la paroi du réacteur tubulaire (6), **caractérisé en ce qu'**une turbulence est produite dans la buse centrale et la turbulence de l'écoulement réalisé dans la buse centrale (5) est en particulier augmentée par l'intermédiaire d'un ou plusieurs éléments encastrés produisant une turbulence (7) et le courant d'éduit contenant les diamines et/ou les triamines étant introduit dans le réacteur tubulaire (6) par l'intermédiaire de la buse centrale (5) et le courant d'éduit contenant le phosgène étant introduit dans le réaction tubulaire (6) par l'intermédiaire de l'espace annulaire (3).

2. Procédé selon la revendication 1, dans lequel le courant d'éduit contenant les diamines et/ou les triamines et le courant d'éduit contenant le phosgène sont permutés de telle sorte que le courant d'éduit contenant les diamines et/ou les triamines est introduit dans le réacteur tubulaire (6) par l'intermédiaire de l'espace annulaire (3) et le courant d'éduit contenant le phosgène est introduit dans le réacteur tubulaire (6) par l'intermédiaire de la buse centrale (5).

3. Procédé selon la revendication 1 ou 2, **caractérisé en ce que** l'on utilise comme éléments produisant une turbulence des plaques inclinées rondes ou angulaires.

4. Procédé selon la revendication 1 ou 2, **caractérisé en ce que** l'on utilise comme élément produisant une turbulence une hélice (7).

5. Procédé selon l'une quelconque des revendications 1 à 4, **caractérisé en ce que** l'on prépare des diisocyanates par phosgénation des diamines correspondantes.

6. Procédé selon l'une quelconque des revendications 1 et 3 à 5, **caractérisé en ce que** l'on introduit dans le réacteur tubulaire (6) des diamines (cyclo)aliphatiques et/ou aromatiques sous forme gazeuse à de 200°C à 600°C par l'intermédiaire de la buse centrale (5) et on introduit du phosgène dans le réacteur tubulaire (6) dans un excès stoechiométrique à de 200°C à 600°C par l'intermédiaire de l'espace annulaire (3).

7. Procédé selon l'une quelconque des revendications 1 à 6, **caractérisé en ce que** l'on utilise de l'isophoronediamine (IPDA) ou de l'hexaméthylènediamine (HDA) ou du bis(p-aminocyclohexyl)méthane comme diamines.

8. Procédé selon l'une quelconque des revendications 1 à 6, **caractérisé en ce que** l'on utilise des mélanges de 2,4/2,6-toluylènediamine des rapports d'isomères 80/20 et 65/35 ou l'isomère de 2,4-toluylènediamine pure comme diamines.

9. Procédé selon l'une quelconque des revendications 1 à 8, **caractérisé en ce que** l'on prépare du triisocyanatononane.
